# DEMANDE DE BREVET EUROPEEN

(11) **EP 0 687 452 A1**
(43) Date de publication de la demande: **20.12.1995**
(21) Numéro de dépôt: 95401386.8
(22) Date de dépôt: 14.06.1995
(51) Int. Cl.: A61F 2/34, A61B 17/17, A61F 2/46

(54) **Prothèse cotyloidienne, son utilisation et instrument ancillaire pour sa mise en place**

(30) Priorité: 16.06.1994 FR 9407391
(71) Demandeur: Labourdette, Patrick Marie Croly, F-59300 Valenciennes (FR)
(72) Inventeur: Labourdette, Patrick Marie Croly, F-59300 Valenciennes (FR)
(74) Mandataire: Beauchamps, Georges

(57) **Abrégé**

La présente invention concerne une prothèse cotyloïdienne.

La prothèse de l'invention est constituée d'une cupule (1) comportant des moyens d'ancrage primaires (3,4) dans le cotyle et présentant extérieurement une forme globalement hémisphérique (2) et intérieurement un logement cylindrosphérique (7), et d'un insert (17) pourvu de moyens de liaison (19) avec la cupule (1) et présentant extérieurement une forme correspondante audit logement (7) dans lequel l'insert (17) est reçu et intérieurement une surface d'appui (20) destinée à recevoir la tête d'une tige de prothèse fémorale, caractérisée en ce qu'elle comprend des moyens élastiques (16) pour engendrer une précontrainte entre le cotyle, la cupule (1) et l'insert (17).

L'invention s'applique notamment à la chirurgie médicale.

## Description

La présente invention concerne une prothèse cotyloïdienne, son utilisation en vue de réduire l'usure de la prothèse et le basculement de celle-ci dans le cotyle, et pour favoriser une repousse osseuse régulière de l'os iliaque, ainsi que l'instrumentation ancillaire pour sa mise en place.

Lors d'une dégénérescence arthrosique ou rhumatismale, d'une ostéonécrose ou d'une fracture du col fémoral, il peut être nécessaire de remplacer l'articulation coxo-fémorale par une prothèse.

Certaines prothèses sont constituées par exemple de deux éléments, à savoir une cupule cotyloïdienne, généralement métallique, à implanter dans la cavité cotyloïdienne osseuse et un insert en matériau peu abrasif, comme une matière plastique telle que le polyéthylène, emboîté dans la cupule métallique, et dans lequel insert est articulée la tête d'une tige de prothèse fémorale implantée quant à elle dans le canal médullaire du fémur.

La fixation de la cupule dans la cavité cotyloïdienne naturelle osseuse d'un individu peut se faire avec ou sans ciment. Dans le cas d'une fixation sans ciment, il est nécessaire de prévoir sur les éléments précités des moyens d'ancrage primaires pour que, dès la mise en place des éléments de prothèse, la fixation de la prothèse dans l'os iliaque adjacent soit suffisante pour permettre à l' individu opéré de reprendre rapidement la marche, de récupérer la mobilité de sa hanche dès le terme de l'opération et de lui permettre aussitôt la remise en charge. L'ancrage secondaire se développera par la suite au cours du temps par repousse osseuse entre la surface de la cupule et l'os adjacent dans lequel elle est implantée pour former une liaison osseuse, ce qui constitue à long terme la fixation de la prothèse.

Il est connu pour favoriser la repousse osseuse et donc améliorer l'ancrage secondaire, de traiter la surface externe de la cupule par un grenaillage au corindon.

Un type de prothèse cotyloïdienne actuellement utilisé comporte par exemple :
- une cupule pourvue de moyens d'ancrage primaires dans la cavité cotyloïdienne et présentant extérieurement une forme globalement hémisphérique et intérieurement un logement sensiblement hémisphérique, et
- un noyau ou insert pourvu de moyens de liaison avec la cupule et présentant extérieurement une forme correspondante au logement précité dans lequel il est reçu et intérieurement une surface d'appui hémisphérique destinée à recevoir la tête d'une tige de prothèse fémorale.

On connaît déjà des moyens d'ancrage primaires de la cupule cotyloïdienne dans le cotyle osseux, qui comportent au moins une vis engagée à travers une ouverture ménagée dans la paroi de ladite cupule.

Ces moyens connus ne permettent pas un ancrage suffisant à la prothèse dans le cotyle car une mobilisation de la cupule cotyloïdienne peut se produire notamment autour de son axe polaire ainsi qu'une migration de la cupule à l'intérieur du cotyle osseux au cours du remodelage que subit l'os coxal dans le temps.

Dans les prothèses actuellement connues, la liaison entre le noyau et la cupule métallique s'effectue généralement par agrafage du noyau sur le bord inférieur de la cupule.

Cette liaison connue présente de nombreux inconvénients résultant notamment de l'usure et/ou du fluage du noyau dans la cupule à la suite de micro-déplacements entre eux, par exemple par rotation ou bascule du noyau dans la cupule, ce qui peut provoquer le détachement de débris de polyéthylène à partir du noyau qui sont susceptibles de provoquer une réponse biologique nuisible aux performances cliniques à long terme, et d'affecter l'adaptation, le positionnement et la stabilité de l'implant tant cotyloïdien que fémoral.

En outre, les prothèses actuellement connues ne comportent pas de moyen assurant une stabilité primaire parfaite de la prothèse dans le cotyle et un basculement de la prothèse peut se produire dans la cavité cotyloïdienne.

On ne connaît pas non plus actuellement de prothèses permettant d'éviter tout micro-déplacement entre le cotyle et la prothèse, ce qui peut provoquer une résorption osseuse et gêner la repousse osseuse régulière.

La qualité de l'ancrage primaire et secondaire de la prothèse dans la cavité cotyloïdienne dépend de la qualité de l'empreinte qui est formée dans le cotyle osseux et des moyens de mise en place de la prothèse dans celui-ci. Toutefois, les instruments médicaux ancillaires connus ne permettent pas d'obtenir une empreinte de bonne qualité dans le cotyle.

La présente invention a donc pour but d'éliminer les inconvénients précités et de proposer une prothèse cotyloïdienne permettant de réduire son usure, son basculement et de favoriser la repousse osseuse.

A cet effet, la présente invention a pour objet une prothèse cotyloïdienne constituée d'une cupule, par exemple métallique, comportant des moyens d'ancrage primaires dans la cavité cotyloïdienne osseuse et présentant extérieurement une forme globalement convexe et intérieurement un logement sensiblement concave, et d'un insert, par exemple en matière plastique, pourvu de moyens de liaison avec la cupule et présentant extérieurement une forme correspondante audit logement dans lequel l'insert est reçu et intérieurement une surface d'appui hémisphérique destinée à recevoir la tête d'une tige de prothèse fémorale, caractérisée en ce qu'elle comprend des moyens élastiques pour engendrer une précontrainte entre le cotyle, la cupule et l'insert.

Dans un mode de réalisation préféré de l'invention, les moyens élastiques précités sont formés d'au moins une fente ménagée à travers la paroi de la cupule à partir du voisinage du pôle de celle-ci jusqu'à son bord périphérique inférieur, suivant un méridien, pour permettre l'expansion et la rétraction de la cupule par rapport à une position intermédiaire de repos.

Selon une autre caractéristique de l'invention, la ou les fentes méridiennes sont ménagées dans seulement une moitié de cupule, à l'opposé de l'autre moitié de cupule comportant les moyens d'ancrage primaires dans le cotyle.

Les moyens élastiques de la prothèse de l'invention établissant une précontrainte entre la prothèse et le cotyle, cette précontrainte pourrait provoquer une résorption osseuse. Par conséquent, il est avantageux de prévoir ces moyens élastiques dans la moitié de cupule destinée à être reçue dans la partie élastique inférieure du cotyle comprenant les cornes.

Selon encore une autre caractéristique de l'invention les moyens d'ancrage primaires comprennent un bossage arqué concentrique avec la cupule et ménagé en saillie sur la surface externe de la cupule, à travers lequel bossage sont ménagés des perçages traversant pour loger des vis de fixation de la cupule dans le cotyle osseux.

La cupule de la prothèse cotyloïdienne peut comporter avantageusement, au niveau de son bord périphérique inférieur, une collerette circulaire destinée à venir s'appuyer contre un logement correspondant formé au bord du cotyle.

Selon une autre caractéristique de l'invention, le logement interne de la cupule présente à partir du bord périphérique inférieur une partie cylindrique qui se prolonge par une partie sphérique tronquée au voisinage du pôle de la cupule par une surface plane et parallèle au plan équatorial de la cupule, pour éviter le basculement du noyau.

La cupule peut comporter en outre sous la collerette précitée au moins un ergot en saillie vers le bas apte à venir se loger dans une encoche de forme correspondante ménagée dans une bordure périphérique entourant la base de l'insert de la prothèse cotyloïdienne, pour empêcher toute rotation de l'insert dans la cupule autour de l'axe polaire.

Selon une autre caractéristique de l'invention, la taille de chaque encoche et de l'ergot associé peuvent être sensiblement différentes, afin d'établir un serrage et un blocage entre la cupule et l'insert.

La présente invention a également pour objet l'utilisation d'une prothèse cotyloïdienne comportant des moyens élastiques de précontrainte pour réduire l'usure entre la cupule et l'insert.

La présente invention vise également l'utilisation d'une prothèse cotyloïdienne comprenant des moyens élastiques de précontrainte pour réduire le fluage du noyau et le détachement de débris de plastique.

L'invention a encore pour objet l'utilisation d'une prothèse cotyloïdienne comportant une collerette circulaire au niveau du plan équatorial de la cupule pour réduire le basculement de celle-ci et la migration de la cupule vers le fond de la cavité cotyloïdienne.

La présente invention vise en outre l'utilisation d'une prothèse cotyloidienne telle que définie précédemment pour réduire les micro-déplacements de la cupule dans le cotyle et favoriser la repousse osseuse.

La présente invention vise enfin une instrumentation ancillaire pour la mise en place de la prothèse cotyloïdienne telle que définie ci-dessus, comportant notamment un gabarit de perçage et un porte-gabarit, caractérisée en ce que le gabarit de perçage est constitué d'une pièce massive cylindrosphérique comportant au moins deux trous ménagés à partir de sa face inférieure, un premier trou débouchant sur la face sphérique externe du gabarit pour creuser un alésage qui correspond au bossage de la cupule cotyloïdienne et qui est obtenu à l'aide d'une fraise passant à travers ledit premier trou, et le second trou traversant axialement le gabarit pour percer un orifice centro-cotyloïdien dans la cavité cotyloïdienne.

Le gabarit de perçage comprend, avantageusement, un troisième trou qui débouche sur la face sphérique externe du gabarit et qui est écarté du premier trou d'un arc de cercle correspondant au bossage de la cupule cotyloïdienne.

Le gabarit de perçage peut comporter en outre un quatrième trou associé à un poussoir et destiné à recevoir et à retenir un téton de fixation du porte-gabarit.

Selon une autre caractéristique de l'invention, l'instrumentation ancillaire comporte une fraise circulaire apte à creuser un logement circulaire dans l'os coxal pour recevoir la collerette circulaire de la cupule cotyloïdienne, ladite fraise étant supportée par un porte-fraise pourvu d'un piston mobile axialement, dont la tige est apte à traverser axialement la fraise circulaire et dont la tête vient se loger dans le troisième trou central du gabarit de perçage précité.

Selon encore une autre caractéristique de l'invention, l'instrumentation ancillaire comprend un porte-cupule qui comporte un manche se prolongeant par une tige jusqu'à un téton de centrage et un moyen de blocage en rotation, autour de l'axe de ladite tige, d'une cupule montée sur ledit téton. Ce moyen de blocage en rotation peut comporter un manchon monté coulissant axialement sur ladite tige, et une patte mortaisée à une extrémité libre et montée, de préférence mobile, à travers la tête du manchon et perpendiculairement à l'axe de la tige et sensiblement au voisinage du téton précité.

On peut prévoir un moyen élastique entre le manche et/ou la tige du porte-cupule et le manchon coulissant pour solliciter ce dernier vers le téton de centrage.

Ce moyen élastique peut être avantageusement un ressort de compression interposé entre la tige et le manchon et prenant appui à une extrémité sur un épaulement radial de la tige et à l'autre extrémité contre la patte précitée pour à la fois bloquer cette dernière en position à travers la tête du manchon et solliciter ce dernier vers le téton précité.

L'invention sera mieux comprise et d'autres buts, caractéristiques, détails et avantages de celle-ci apparaîtront plus clairement au cours de la description explicative qui va suivre d'un mode de réalisation particulier actuellement préféré de l'invention, donné uniquement à titre d'exemple illustratif et non limitatif, en référence aux dessins schématiques annexés, dans lesquels :

La figure 1 est une vue en perspective de la cupule de prothèse cotyloïdienne de l'invention.

La figure 2 est une vue en coupe de la cupule de la figure 1, suivant un méridien de celle-ci.

La figure 3 est une vue en perspective du noyau d'une prothèse cotyloïdienne de l'invention.

La figure 4 est une vue de dessous du noyau de la figure 3.

La figure 5 est une vue de dessus, partiellement arrachée, d'un gabarit de perçage associé à un porte-gabarit de l'instrumentation ancillaire selon l'invention.

La figure 6 est une vue en coupe du gabarit de perçage associé au porte-gabarit de la figure 5, suivant la ligne VI-VI.

La figure 7 est une vue en coupe et partiellement éclatée d'un pion de centrage et du gabarit de perçage de la figure 5, associé à une fraise flexible, suivant la ligne VII-VII.

La figure 8 est une vue latérale d'une fraise circulaire de l'instrumentation ancillaire selon l'invention.

La figure 9 est une vue latérale et partiellement en coupe d'un porte-fraises de l'instrumentation ancillaire de l'invention, destiné à porter la fraise circulaire de la figure 8.

La figure 10 est une vue latérale d'un porte-cupule de l'instrumentation ancillaire de la présente invention.

La figure 11 est une vue partielle et en coupe axiale, suivant la ligne XI-XI de la figure 12, de la cupule de l'invention associée au porte-cupule de la figure 10.

La figure 12 est une vue en coupe du porte-cupule, suivant la ligne XII-XII de la figure 11.

Les figures 13 et 14 sont des vues latérales respectivement d'un impacteur de cupule et d'un porte-noyau.

La figure 15 est une vue latérale d'un manche universel destiné à supporter l'impacteur de cupule de la figure 11 et le porte-noyau de la figure 12.

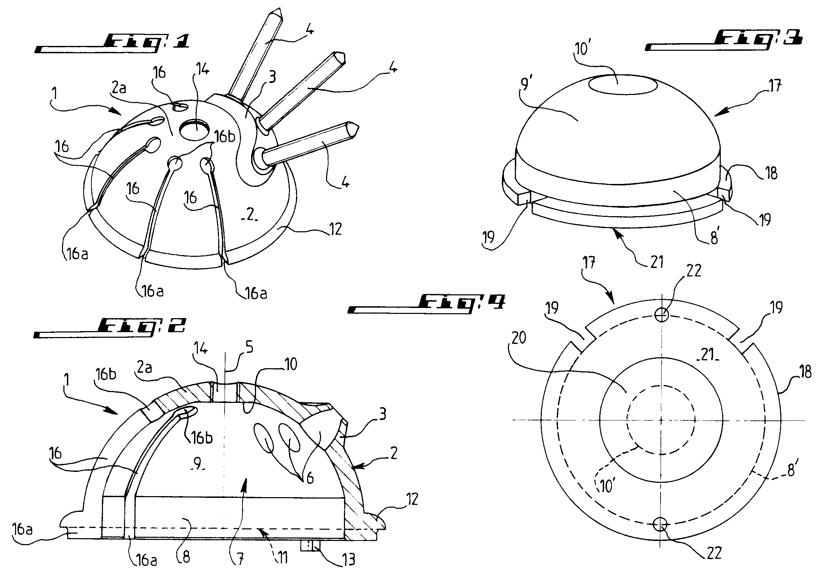

Suivant l'exemple de réalisation représenté sur la figure 1, la prothèse cotyloïdienne de l'invention comporte une cupule creuse 1 dont la surface externe 2 est globalement hémisphérique.

Un bossage 3 en forme d'arc circulaire fait saillie de la surface externe 2 radialement par rapport au centre de la sphère délimitant la cupule 1.

L'arc de cercle du bossage 3 s'étend sur un angle important de l'ordre de 120°, ce qui permet de réaliser un excellent ancrage primaire de la cupule 1 dans la cavité cotyloïdienne, par l'intermédiaire de trois vis 4 traversant ledit bossage 3.

Ce bossage permet en outre d'éviter tout mouvement de rotation de la cupule 1 autour de son axe polaire 5.

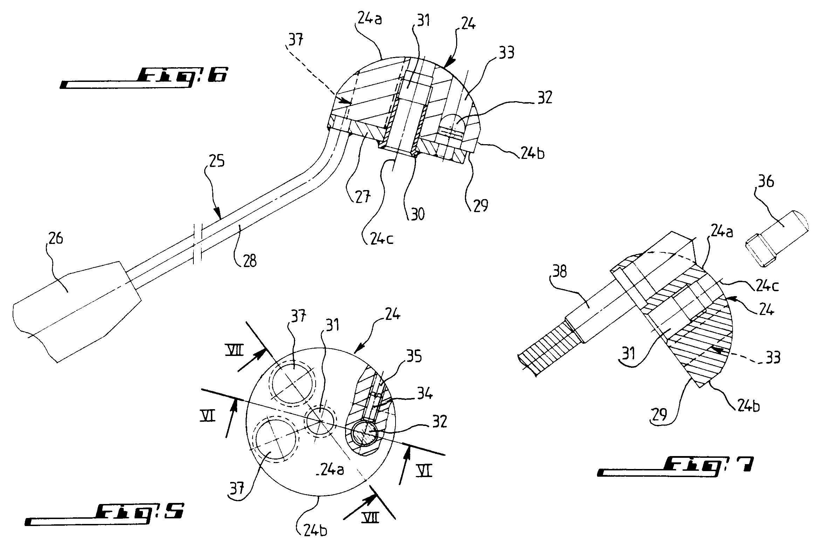

On voit sur la figure 2 que la tête de chaque vis 4 est destinée à être reçue dans des perçages respectifs 6 qui débouchent dans le logement interne 7 de la cupule 1.

Les perçages 6 présentent une forme interne légèrement concave de manière que la tête des vis 4 puisse rotuler à l'intérieur de son perçage respectif 6 pour permettre au chirurgien d'orienter les vis 4 suivant une direction adaptée à la hanche (lignes de force) où la prothèse doit être implantée.

Le logement interne 7 de la cupule 1 présente une partie inférieure cylindrique 8 qui se prolonge par une partie sphérique 9 et se termine à son sommet par une partie plane 10 parallèle au plan équatorial 11 de la cupule 1.

Une collerette arrondie et circulaire 12 fait saillie extérieurement du bord périphérique inférieur ou du plan équatorial 11 de la cupule 1.

Sous la collerette périphérique 12 fait saillie vers le bas au moins un ergot 13.

Un taraudage 14 traverse la paroi de la cupule 1 perpendiculairement à la partie plane 10 du logement 7 pour recevoir l'extrémité filetée extérieurement 15a d'un porte-cupule 15, représenté sur la figure 10.

La paroi de la cupule 1 présente en outre une pluralité de fentes 17 ménagées suivant des méridiens de la cupule 1, à partir du voisinage du pôle 2a de la cupule 1 jusqu'à la collerette circulaire 12.

Ces fentes méridiennes 16 ont leurs extrémités inférieures 16a ouvertes et leurs extrémités supérieures 16b fermées et en forme de cercle au voisinage du pôle 2a.

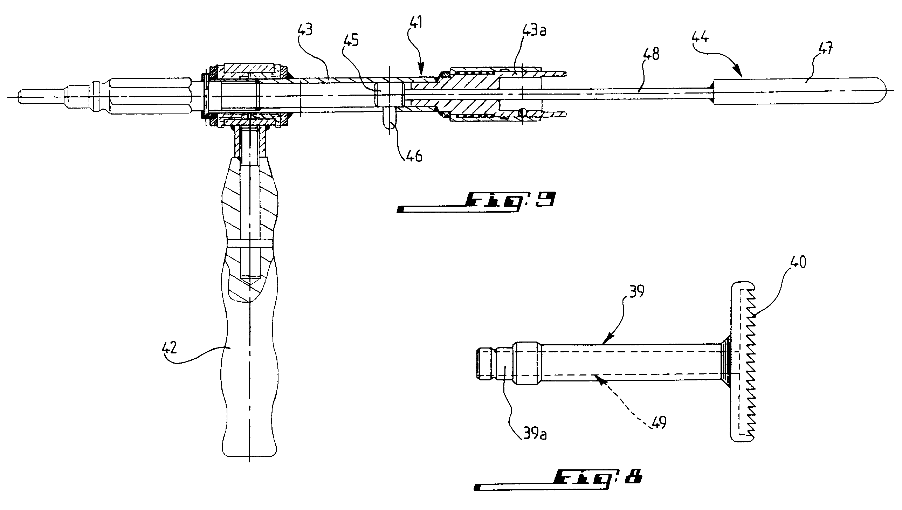

La prothèse cotyloïdienne de l'invention comporte en outre un insert au noyau 17 (voir figure 3), par exemple en polyéthylène, présentant une surface externe de forme correspondante au logement 7, à savoir une partie cylindrique 8', une partie sphérique 9' et une partie plane 10'.

La forme particulière du logement 7 et de la surface externe du noyau 17 empêche toute rotation de celui-ci dans la cupule 1 par rapport à un axe perpendiculaire à l'axe polaire 5.

Le noyau 17 comporte en outre une bordure périphérique inférieure 18 qui vient sous la collerette circulaire 12 de la cupule 1, avec un faible jeu.

Des encoches 19 sont formées à travers la bordure 18 qui s'étend dans un plan sensiblement équatorial, pour recevoir les ergots 13 de la cupule 1, ce qui empêche toute rotation relative du noyau 17 dans la cupule 1 par rapport à l'axe polaire 5, et qui permet en outre la retenue du noyau 17.

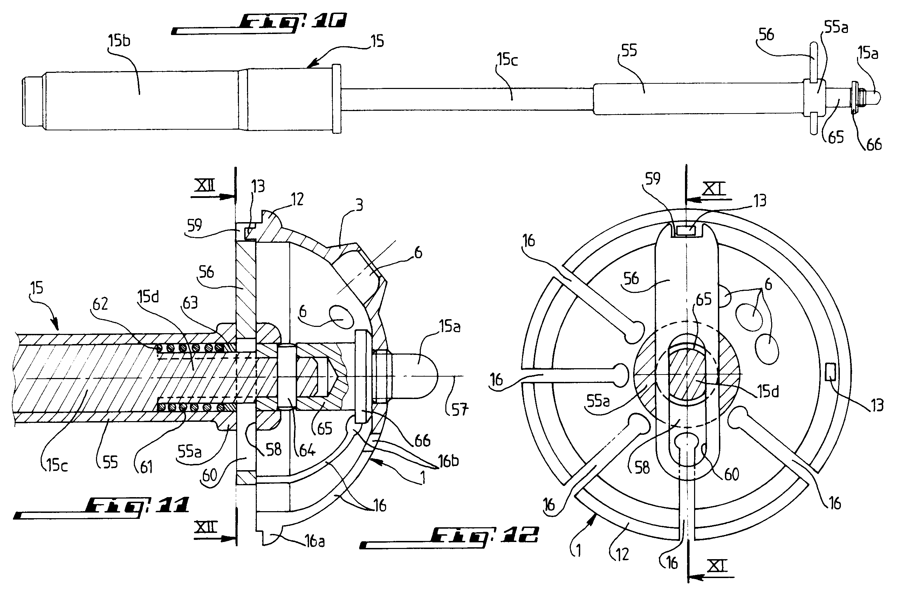

On voit sur la figure 4 que le noyau 17 comporte un logement hémisphérique 20 ménagé à partir de la face inférieure 21 du noyau 17, pour recevoir la tête d'une tige fémorale non représentée.

Deux évidements 22 sont ménagés diamétralement dans le noyau 17 à partir de sa face inférieure 21 pour recevoir deux tétons de fixation 23a d'un porte-noyau 23 illustré sur la figure 12.

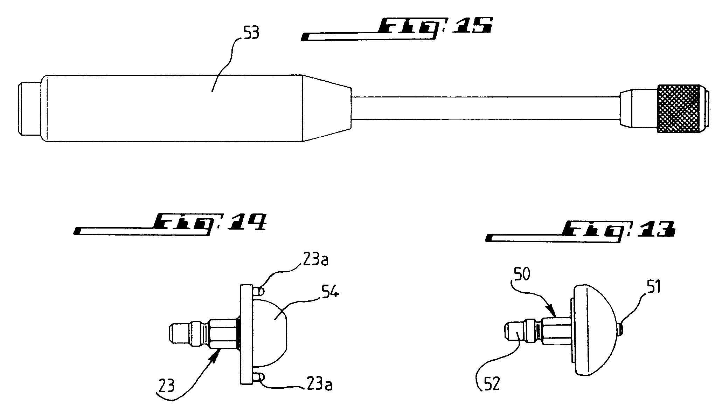

On va maintenant décrire la technique opératoire de mise en place de la prothèse cotyloïdienne de l'invention en référence à l'instrumentation ancillaire de l'invention illustrée sur les figures 5 à 13.

Après une préparation chirurgicale connue, le chirurgien effectue un alésage du cotyle à l'aide d'une fraise en forme de râpe hémisphérique. L'alésage du cotyle s'effectue en éliminant toute trace de cartilage et jusqu'à l'obtention d'un os saignant.

Le chirurgien introduit ensuite le gabarit de perçage 24 de l'invention dans la cavité cotyloïdienne (non représentée) à l'aide d'un porte-gabarit 25 qui est représenté partiellement interrompu sur la figure 6.

Le porte-gabarit 25 comporte un manche 26 relié à une plaque de support 27 par l'intermédiaire d'une tige médiane 28.

La tige médiane 28 est fixée à la périphérie de la face inférieure de la plaque de support 27, tandis que la face supérieure de la plaque 27 vient s'appuyer contre la face plane inférieure 29 du gabarit de perçage 24.

Le gabarit de perçage 24 présente une surface externe globalement sphérique 24a séparée de sa face plane inférieure 29 par une partie cylindrique 24b.

La courbure de la surface externe sphérique 24a est sensiblement la même, voire légèrement inférieure à la courbure de la surface externe 2 de la cupule 1 de la prothèse cotyloïdienne de l'invention.

La plaque 27 comporte axialement un manchon 30 qui fait saillie à partir de la face supérieure de la plaque 27 pour venir s'emboîter dans un trou 31 traversant axialement le gabarit de perçage 24.

La plaque 27 comporte en outre un téton 32 radialement espacé du manchon 30 qui fait également saillie à partir de la face supérieure de la plaque 27 de manière à être reçu dans un autre trou 33 ménagé à travers le gabarit 24, parallèlement au trou 31.

Le trou 33 est représenté traversant sur les dessins, mais il peut en variante être borgne.

Le téton 32 présente une forme globalement en champignon ou en altère de sorte que sa partie médiane de section réduite coopère avec un poussoir 34 (représenté sur la figure 5) pour venir bloquer le téton 32 dans le trou du gabarit de perçage 24.

Le poussoir 34 vient en engagement contre le téton 32 par vissage de ce poussoir dans un taraudage 35 ménagé radialement par rapport au trou 33.

La partie supérieure du trou 31 est filetée intérieurement pour recevoir un pion de centrage 36 représenté sur la figure 7 en position démontée.

Le gabarit de perçage 24 comporte deux autres trous traversants 37, filetés intérieurement, espacés radialement du trou traversant 31 et d'axes parallèles à ce dernier.

Ces trous traversants 37 sont destinés à recevoir la tête d'une fraise flexible 38 en forme de mèche représentée sur la figure 7.

Les deux trous traversants 37 sont écartés l'un de l'autre suivant un arc de cercle correspondant au bossage 3 de la cupule 1.

Une fois le gabarit de perçage 24 introduit dans la cavité cotyloïdienne, le chirurgien, tout en maintenant le gabarit de perçage 24 avec le porte-gabarit 25, perce un orifice centro-cotyloïdien dans la cavité cotyloïdienne à l'aide d'un foret de perçage (non représenté) traversant le manchon 30 du porte-gabarit 25 et le trou 31 du gabarit 24.

Après perçage de cet orifice centro-cotyloïdien, le chirurgien visse le pion de centrage 36 dans le trou 31 du gabarit 24, lequel pion 36 vient se loger dans l'orifice centro-cotyloïdien précité.

Le chirurgien introduit alors la fraise flexible 38 dans l'un des trous 37 pour effectuer un premier forage partiel du cotyle, en s'aidant du manche du porte-gabarit 25 pour l'orienter dans l'espace.

Une fois réalisé ce premier forage, le chirurgien introduit alors la fraise flexible 38 dans le second trou 37 pour former un second forage dans le cotyle. Il est possible de maintenir le gabarit 24 immobile en rotation autour de son axe polaire 24c en introduisant une pointe dans le premier forage, pendant que l'on forme le second forage.

Puis, le chirurgien réalise un alésage de forme correspondante au bossage 3 de la cupule 1 en faisant pivoter le gabarit 24 autour de son axe 24c, pour amener la fraise flexible 38 du second forage vers le premier forage.

On obtient ainsi un alésage en arc de cercle pour recevoir le bossage 3.

Le chirurgien retire ensuite le porte-gabarit 25.

Le chirurgien effectue ensuite la résection des ostéophytes qui débordent autour du gabarit 24 et le forage d'un logement circulaire périphérique à l'entrée du cotyle à l'aide d'une fraise circulaire dite de forme 39 représentée sur la figure 8.

La fraise circulaire 39 présente à une extrémité une couronne dentée 40 qui est guidée en sa périphérie interne par la partie cylindrique 24b du gabarit 24 et qui vient s'adapter autour de cette partie 24b pour aléser le logement précité.

Le profil des dents de la fraise 39 est identique au profil de la collerette 12 de la cupule 1.

La fraise circulaire 39 est supportée par un porte-fraise 41 représenté sur la figure 9.

Le porte-fraise 41 présente la forme générale d'un "pistolet" comportant un manche 42 surmonté d'un tube de guidage 43 perpendiculaire audit manche 42.

Le tube de guidage 43 retient à l'une 43a de ses extrémités l'autre extrémité 39a de la fraise circulaire 39, à l'opposé de l'extrémité ayant la couronne dentée 40.

Le porte-fraise 41 comporte en outre un piston de centrage 44 constitué d'un élément 45 mobile axialement à l'intérieur du tube de guidage 43 et actionné manuellement par un doigt 46 qui fait saillie hors du tube de guidage 43, d'une tête de piston 47 qui fait saillie axialement hors du tube 43 et d'une tige de piston 48 reliant ladite tête 47 audit élément mobile 45.

Lorsque la fraise 39 est montée sur l'extrémité 43a du porte-fraise 41, la tête de piston 47 traverse axialement la fraise 39 par l'intermédiaire d'un alésage 49, pour venir se loger dans le trou 31 du gabarit 24.

Le piston 44 assure donc un préguidage axial de la fraise 39 lors de sa phase d'approche, la fraise 39 étant ensuite guidée par la partie cylindrique 24b du gabarit 24.

Le fait de prévoir le piston 44 coulissant assure une approche correcte de la fraise 39, même lorsque des ostéophytes gênent cette approche et débordent sur le gabarit 24.

Le chirurgien retire ensuite le gabarit de perçage 24 et insère à force la cupule 1 dans l'empreinte ainsi formée dans le cotyle osseux, à l'aide du porte-cupule 15 représenté sur la figure 10.

Le porte-cupule 15 comporte un manche 15b se prolongeant par une tige allongée 15c jusqu'au téton de centrage 15a sur lequel vient se visser le taraudage 14 de la cupule 1.

Le porte-cupule 15 comporte en outre un manchon 55 monté coulissant axialement sur la tige allongée 15c, la tête 55a du manchon 15 étant traversée par une patte mortaisée 56 qui est mobile dans cette tête 55a dans une direction perpendiculaire à l'axe 57 de la tige 15c.

La patte 56 se déplace dans un logement 58 ménagé diamétralement dans la tête 55a et comporte à une extrémité libre longitudinale une mortaise 59, par exemple en forme de U, qui est de forme sensiblement adaptée aux ergots 13 de la cupule 1 de l'invention.

La patte 56 comporte en outre un trou oblong traversant 60 à travers lequel passe l'extrémité 15d de la tige allongée 15c.

La patte 56 et le manchon 55 sont donc mobiles axialement le long de l'extrémité 15d de la tige 15c.

Un ressort de compression hélicoïdal 61 est monté axialement autour de l'extrémité 15d de la tige 15c et il est interposé entre ladite extrémité 15d et la paroi interne du manchon 55.

Ce ressort de compression 61 prend appui à une spire d'extrémité sur un épaulement radial 62 qui sépare le corps principal de la tige 15c de son extrémité 15d et à l'autre spire d'extrémité contre la patte 56, par l'intermédiaire par exemple d'une rondelle d'appui 63, pour venir bloquer la patte 56 en position à travers la tête 55a du manchon 55 par pression et frottement.

Bien entendu, on pourrait prévoir en variante tout autre moyen élastique adapté pour remplacer le ressort hélicoïdal de compression 61.

Le fait de prévoir la patte 56 mobile transversalement et le manchon 55 mobile axialement permet d'adapter le porte-cupule 15 à différentes tailles de cupule 1, selon respectivement son diamètre équatorial et sa hauteur axiale.

L'assemblage de la mortaise 59 de la patte 56 avec un ergot 13 peut s'effectuer aisément de la manière suivante : après avoir vissé le téton 15a dans le taraudage 14 de la cupule 1, on exerce d'abord une traction sur le manchon 55 pour éloigner celui-ci du téton de centrage 15a et on relâche ensuite le manchon 55, après avoir positionné correctement la patte 56 à travers son logement 58, pour qu'il vienne s'emboîter dans la direction axiale 57 avec l'ergot associé 13.

La mortaise 59 de la patte 56 coopérant avec un ergot 13, on empêche ainsi toute rotation, autour de l'axe 57 ou 5, de la cupule 1 par rapport au porte-cupule 15.

Cette caractéristique est particulièrement avantageuse car elle évite tout risque de dévissage du téton de centrage 15a par rapport à la cupule 1, lorsque le chirurgien veut faire pivoter la cupule 1 dans le sens de dévissage du téton 15a et que la cupule 1 est insérée dans la cavité cotyloïdienne.

On voit sur la figure 11 que la tige 15c est assemblée au téton de centrage 15a par l'intermédiaire d'une goupille 64 qui traverse diamétralement l'extrémité 15d et une pièce distincte 65 solidaire du téton 15a, pour permettre un montage et un démontage aisés du ressort 61 entre l'épaulement 62 et la patte 56.

Une collerette d'appui 66 sépare le téton fileté 15a du corps principal de la pièce 65 pour s'appuyer contre la partie plane 10 de la cupule 1.

Le positionnement correct de la cupule 1 dans l'empreinte formée dans le cotyle s'effectue en insérant l'extrémité saillante du téton 15a dans l'orifice centro-cotyloïdien et le bossage 3 de la cupule 1 dans son alésage de réception.

L'empreinte formée dans le cotyle présente une sphéricité légèrement inférieure à celle de la cupule 1, de sorte que la cupule 1 se rétracte lors de son insertion dans l'empreinte grâce à l'élasticité fournie par les fentes méridiennes 16.

Le chirurgien retire ensuite le porte-cupules 15 et utilise un impacteur de cupule 50 pour effectuer l'ajustement final de la cupule 1 dans le cotyle osseux (voir figure 13).

L'impacteur du cupule 50 comporte également un pion de centrage 51 destiné à venir se visser dans le taraudage 14 de la cupule 1 et présente une forme extérieure sensiblement correspondante au logement 7 de la cupule 1.

L'impacteur de cupule 50 est supporté à son extrémité 52 par un manche universel 53 représenté sur la figure 15.

La mise en place définitive de la cupule 1 à l'intérieur du cotyle osseux s'effectue alors en martelant l'extrémité du manche universel 53.

Il est préférable d'effectuer l'installation définitive de la cupule 1 à l'aide de l'impacteur 50, plutôt qu'avec le porte-cupule 15, car dans ce dernier cas, la liaison entre le téton 15a et le taraudage 14 de la cupule 1 pourrait ne pas être suffisamment résistante pour supporter le martèlement précité.

Le chirurgien effectue ensuite de manière classique des trous de forage dans la cavité cotyloïdienne, à travers les trous 6 de la cupule 1, pour l'installation des vis 4 de fixation.

Le noyau 17 est enfin ajusté à force dans la cupule 1, à l'aide du porte-noyau 23 (représenté sur la figure 14) qui supporte le noyau 17 par l'intermédiaire de deux pions 23a qui viennent se loger dans les évidements 22 du noyau 17 et d'un bossage hémisphérique 54 de forme correspondante au logement interne hémisphérique 20 du noyau 17.

La cupule 1 ayant subi une rétractation lors de son ajustement dans le cotyle osseux, la cupule 1 subit maintenant une expansion lors de l'ajustement du noyau 17 dans le logement 7 de la cupule 1, ce qui engendre une précontrainte entre, d'une part, la partie inférieure élastique du cotyle et une moitié de la cupule 1 et, d'autre part, la cupule 1 et le noyau 17.

Bien que la présente invention ait été décrite en liaison avec un mode de réalisation particulier, il est évident qu'elle n'y est nullement limitée et qu'on peut lui apporter de nombreuses variantes et modifications sans pour autant sortir de son cadre ni de son esprit.

## Revendications

1. Prothèse cotyloïdienne constituée d'une cupule (1), par exemple métallique, comportant des moyens d'ancrage primaires (3, 4) ménagés en saillie de la surface externe (2) de la cupule, à travers lesquels moyens d'ancrage primaires sont ménagés des perçages traversants (6) pour loger des vis (4) de fixation de la cupule dans la cavité cotyloïdienne osseuse, la cupule (1) présentant extérieurement une forme globalement convexe (2) et intérieurement un logement sensiblement concave (7), et d'un insert (17), par exemple en matière plastique, pourvu de moyens de liaison (19) avec la cupule et présentant extérieurement une forme correspondante audit logement (7) dans lequel l'insert (17) est reçu et intérieurement une surface d'appui hémisphérique (20) destinée à recevoir la tête d'une tige de prothèse fémorale, ladite prothèse comprenant des moyens élastiques (16) pour engendrer une précontrainte entre le cotyle, la cupule (1) et l'insert (17), les moyens élastiques précités étant formés d'au moins une fente (16) ménagée à travers la paroi de la cupule (1) à partir du voisinage du pôle (2a) de celle-ci jusqu'à son bord périphérique inférieur (12), suivant un méridien, pour permettre l'expansion et la rétraction de la cupule (1) par rapport à une position intermédiaire de repos, la ou les fentes méridiennes (16) étant ménagées dans seulement une moitié de cupule (1), à l'opposé de l'autre moitié de cupule comportant les moyens d'ancrage primaires (3, 4) dans le cotyle, caractérisée en ce que les moyens d'ancrage primaires sont constitués d'un bossage arqué (3) concentrique avec la cupule (1) et faisant saillie de la surface externe de la cupule (1).

2. Prothèse cotyloïdienne selon la revendication 1, caractérisée en ce que le bossage précité (3) forme un arc de cercle s'étendant sur environ 120°, pour recevoir par exemple trois vis de fixation (4).

3. Prothèse cotyloïdienne selon la revendication 1 ou 2, caractérisée en ce que le logement interne (7) de la cupule (1) présente à partir du bord périphérique inférieur (12) une partie cylindrique (8) qui se prolonge par une partie sphérique (9) tronquée au voisinage du pôle (2a) de la cupule (1) par une surface plane (10) parallèle au plan équatorial (11) de la cupule (1).

4. Prothèse cotyloïdienne selon l'une quelconque des revendications précédentes, caractérisée en ce que la cupule (1) comporte au niveau de son bord périphérique inférieur, une collerette circulaire (12) destinée à venir s'appuyer contre un logement correspondant formé au bord du cotyle.

5. Prothèse cotyloïdienne selon la revendication 4, caractérisée en ce que la cupule (1) comporte en outre sous la collerette (12), au moins un ergot en saillie vers le bas (13) apte à venir se loger dans une encoche de forme correspondante (19) ménagée dans une bordure périphérique (18) entourant la base de l'insert (17) de la prothèse cotyloïdienne.

6. Prothèse cotyloïdienne selon la revendication 5, caractérisée en ce que la taille de chaque encoche (19) et de l'ergot associé (13) sont sensiblement différentes afin d'établir un serrage entre la cupule (1) et l'insert (17).

7. Utilisation d'une prothèse cotyloïdienne (1, 17) selon l'une quelconque des revendications précédentes, comportant des moyens élastiques de précontrainte (16) pour réduire l'usure entre la cupule (1) et l'insert (17).

8. Utilisation d'une prothèse cotyloïdienne (1, 17) selon l'une quelconque des revendications 1 à 6, comprenant des moyens élastiques de précontrainte (16) pour réduire le fluage de l'insert (17) et le détachement de débris plastique.

9. Utilisation d'une prothèse cotyloïdienne (1, 17) selon l'une quelconque des revendications 3 à 5, comportant une collerette circulaire (12) au niveau du plan équatorial (11) de la cupule (1) pour réduire le basculement de celle-ci et la migration de la cupule (1) vers le fond de la cavité cotyloïdienne.

10. Utilisation d'une prothèse cotyloïdienne (1, 17) selon l'une quelconque des revendications 1 à 6 pour réduire les micro-déplacements de la cupule (1) dans le cotyle et favoriser la repousse osseuse.

11. Instrumentation ancillaire pour la mise en place de la prothèse cotyloïdienne selon l'une quelconque des revendications 1 à 6, comportant notamment un porte-gabarit (25) et un gabarit de perçage (24) constitué d'une pièce massive comportant au moins un trou (31, 37) ménagé à partir de sa face inférieure (29), le premier trou (37) débouchant sur la face sphérique externe (24a) du gabarit (24) pour creuser un alésage qui correspond aux moyens d'ancrage primaires (3) de la cupule cotyloïdienne (1), et qui est obtenu à l'aide d'une fraise (38) passant à travers ledit premier trou (37), caractérisée en ce que le gabarit de perçage (24) présente une surface externe cylindro-sphérique (24a, 24b) et comporte un second trou (31) traversant axialement le gabarit (24) pour percer un orifice centro-cotyloïdien dans le cotyle.

12. Instrumentation ancillaire selon la revendication 11, caractérisée en ce que le gabarit de perçage comprend un troisième trou (37) qui débouche sur la face sphérique externe (24a) du gabarit (24) et qui est écarté du premier trou (37) d'un arc de cercle correspondant au bossage (3) de la cupule cotyloïdienne (1).

13. Instrumentation ancillaire selon la revendication 11 ou 12, caractérisée en ce que le gabarit de perçage (24) comporte un autre trou (33) associé à un poussoir (34) et destiné à recevoir et à retenir un téton (32) de fixation du porte-gabarit (25).

14. Instrumentation ancillaire selon la revendication 4 et l'une quelconque des revendications 11 à 13, caractérisée en ce qu'elle comporte une fraise circulaire (39) apte à creuser un logement circulaire dans l'os coaxial pour recevoir la collerette circulaire (12) de la cupule cotyloïdienne (1), ladite fraise (39) étant supportée par un porte-fraise (41) pourvu d'un piston (44) mobile axialement, dont la tige (48) est apte à traverser axialement la fraise circulaire (39) et dont la tête (47) vient se loger dans le second trou central (31) du gabarit de perçage (24), et en ce que la partie cylindrique (24b) du gabarit de perçage (24) sert de guide à la couronne dentée (40) de la fraise (39).

15. Instrumentation ancillaire selon l'une quelconque des revendications 11 à 14 ou pour la mise en place de la prothèse cotyloïdienne selon la revendication 5 ou 6, du type comportant un porte-cupule (15) qui comprend un manche (15b) se prolongeant par une tige (15c, 15d) jusqu'à un téton de centrage (15a) et un moyen (55, 56) de blocage en rotation, autour de l'axe (57) de ladite tige, d'une cupule (1) montée sur ledit téton, caractérisée en ce que le moyen de blocage en rotation comporte un manchon (55) monté coulissant axialement sur ladite tige, et une patte (56) mortaisée à une extrémité libre (59) pour s'emboîter avec un ergot (13) de la périphérie (12) de la cupule (1) et montée, de préférence mobile, à travers la tête (55a) du manchon (55) et perpendiculairement à l'axe (57) de la tige précitée, et en ce que le téton (15a) précité est destiné à être vissé sur un taraudage central (14) de la cupule (1).

16. Instrumentation ancillaire selon la revendication 15, caractérisée en ce qu'elle comprend un moyen élastique (61) entre le manche (15b) et/ou la tige (15c, 15d) du porte-cupule (15) et le manchon coulissant (55) pour solliciter ce dernier vers le téton de centrage (15a).

17. Instrumentation ancillaire selon la revendication 16, caractérisée en ce que le moyen élastique est un ressort de compression (61) interposé entre la tige (15d) et le manchon (55) et prenant appui à une extrémité sur un épaulement radial (62) de la tige (15c) et à l'autre extrémité contre la patte précitée (56) pour à la fois bloquer cette dernière en position à travers la tête (55a) du manchon (55) et solliciter ce dernier vers le téton (15a).
